# EUROPEAN PATENT SPECIFICATION

(11) **EP 1 844 776 B1**
(45) Date of publication and mention of the grant of the patent: **28.04.2010**
(21) Application number: 06712759.7
(22) Date of filing: 01.02.2006
(51) Int. Cl.: A61K 31/422, A61K 31/4245, A61P 31/22

(54) **AGENT FOR PREVENTION/TREATMENT OF DISEASE CAUSED BY ACYCLOVIR-RESISTANT HERPESVIRUS**
WIRKSTOFF ZUR PRÄVENTION/BEHANDLUNG VON ERKRANKUNGEN INFOLGE VON ACYCLOVIR-RESISTENTEM HERPESVIRUS
AGENT DESTINE A LA PREVENTION/AU TRAITEMENT D'UNE MALADIE CAUSEE PAR UN VIRUS DE L'HERPES ACYCLOVIR-RESISTANT

(30) Priority: 02.02.2005 JP 2005027106
(43) Date of publication of application: 17.10.2007
(73) Proprietor: Astellas Pharma Inc., Tokyo 103-8411 (JP)
(72) Inventor: SUZUKI, Hiroshi, 2-chome, Chuo-ku Tokyo, 1038411 (JP); CHONO, Koji, 2-chome, Chuo-ku Tokyo, 1038411 (JP); SUDO, Kenji, 2-chome, Chuo-ku Tokyo, 1038411 (JP)
(74) Representative: Bates, Philip Ian
(86) International application number: PCT/JP2006/301616
(87) International publication number: WO 2006/082822

(56) References cited:
- EP-A- 1 340 750
- WO-A-2005/014559
- WO-A1-02/38554
- WO-A1-03/095435
- WO-A1-2005/014559
- CRUTE J J ET AL: "Herpes simplex virus helicase-primase inhibitors are active in animal models of human disease" NATURE MEDICINE, NATURE PUBLISHING GROUP, NEW YORK, NY, US, vol. 8, no. 4, 1 April 2002 (2002-04-01), pages 386-391, XP002969973 ISSN: 1078-8956

## Description

### Technical Field

This invention relates to an agent for preventing or treating diseases caused by acyclovir (ACV)-resistant herpesviruses, which comprises a novel tetrahydro-2H-thiopyran-4-carboxamide derivative as an active ingredient.

### Background Art

Viruses belonging to the Herpesviridae family cause various infectious diseases in human and animals. For example, it is known that varicella zoster virus (VZV) causes varicella and herpes zoster, and herpes simplex virus type 1 and 2 (HSV-1 and HSV-2) cause infections such as herpes labialis, genital herpes, etc. Currently, nucleic acid-based medicaments, such as acyclovir (ACV) and its prodrug, valacyclovir (VCV), and famciclovir (FCV) which is a prodrug of penciclovir, etc., are used as medicaments against herpesviruses such as VZV and HSV. Up to now, it is reported that the developing frequency of resistant viruses against these anti-herpesvirus agents is 1% or less in the patients having normal immune system. However, the developing frequency of resistant viruses is increased to 5 to 30% in the patients who became a state of immunocompromise due to cancer, AIDS, organ transplantation and the like, thus causing a problem from a clinical point of view (Journal of Clinical Virology, 26, 29 - 37, 2003). A further problematic point is that since these resistant herpesviruses gain cross resistance to all of ACV, VCV and FCV, every agent loses its therapeutic effect. Since nucleic acid-based anti-herpesvirus agents Vidarabine and Foscarnet do not always show cross resistance to these resistant herpesviruses, they can be used as substitutive therapeutic agents in some cases, but have causing problems because reduction of blood cells, renal function disorder and the like side effects are observed at a high frequency.
The present inventors previously found an amide compound substituted with a thiazolylphenylcarbamoylmethyl group and with favorable anti-herpesvirus activity, as represented by the following formula where the nitrogen atom of the amide group is substituted directly with an aromatic group aryl or heteroaryl group as ring A, or the salt thereof. Thus, the inventors filed a patent application (Patent Reference 1 and Patent Reference 2). 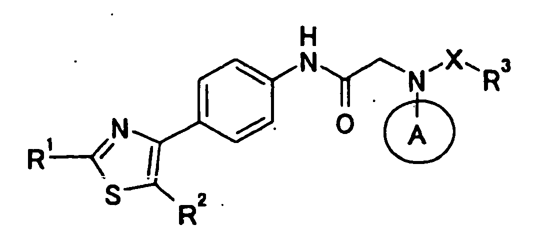 (In the formula, R¹ and R² represent -H, -lower alkyl, -NRaRb or the like; A represents -aryl which may have a substituent(s), -heteroaryl which may have a substituent(s) or the like; X represents CO or SO₂; R³ represents -aryl which may have a substituent(s), -heterocycle which may, for example, have a substituent(s) see the Publication for details).
In addition, a compound represented by the following formula is disclosed in an application by the instant applicant and the like (Patent Reference 3) which was published after the priority date of the instant application. 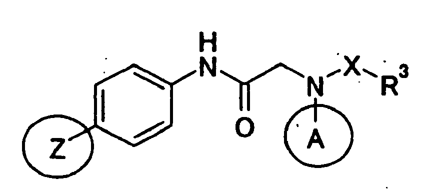 (In the formula, Z represents 1,2,4-oxadiazol-3-yl, 4-oxazolyl or the like, A represents an aryl group or the like which may have substituent(s), X represents CO or SO₂, and R³ represents a heterocycle which may, for example, have substituent(s). See the Publication for details.)
However, nothing is disclosed about the activity of these compounds against ACV-resistant viruses.

Patent Reference 1: International Publication No. 02/38554
Patent Reference 2: International Publication No. 03/95435
Patent Reference 3: International Publication No. 05/014559 Crute JJ et AL: "Herpes simplex virus helicase-primase inhibitors are active in animal models of human disease", Nature Medecine, 8 (4), 386-391, 2002 discloses helicase-primase inhibitors which inhibit the growth of acyclovir-resistant viruses.

### Disclosure of the Invention

### Problems that the Invention is to Solve

Concern has been directed toward the development of a new type preventive or therapeutic agent for diseases caused by ACV-resistant herpesviruses.

### Means for Solving the Problems

The present inventors have conducted extensive studies on compounds having anti-herpesvirus activity and, as a result, unexpectedly found that novel tetrahydro-2H-thiopyran-4-carboxamide derivatives which are characterized in that 1,2,4-oxadiazol-3-yl or 4-oxazolyl is introduced as the Z ring instead of the conventional amino-substituted thiazole ring, as shown in the following general formula (I), have an excellent anti-herpesvirus activity. By further finding that these compounds have an excellent anti-herpesvirus activity against ACV-resistant herpesviruses, the invention has been accomplished.

That is, the invention relates to a medicament for prevention or treatment of diseases associated with ACV-resistant herpesviruses, which comprises an N-{2-[(4-substituted phenyl)amino]-2-oxoethyl}tetrahydro-2H-thiopyran-4-carboxamide compound represented by the following general formula (I) as an active ingredient.

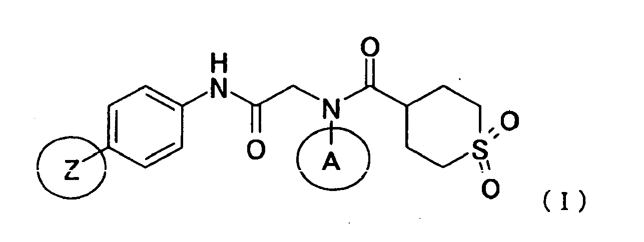

(Symbols in the formula have the following meanings
Z: a 1,2,4-oxadiazol-3-yl or 4-oxazolyl group,
A: a phenyl group which is substituted by at least one methyl group and may further have 1 or 2 substituents selected from the group consisting of a methyl group and halogen atoms, or a 5-indanyl group. The same shall apply hereinafter.)

Particularly, the following compounds are desirable as the compounds represented by the general formula (I), which are the active ingredient of the medicament of the present invention.
(1) A compound in which Z is a 1,2,4-oxadiazol-3-yl group.
(2) A compound in which Z is a 4-oxazolyl group.
(3) A compound in which A is a phenyl group which is substituted by at least one methyl group and may further have 1 or 2 substituents selected from the group consisting of a methyl group and halogen atoms.
(4) A compound in which A is a 5-indanyl group.
(5) A compound selected from the group consisting of
   N-(2,6-dimethylphenyl)-N-(2-{[4-(1,3-oxazol-4-yl)phenyl]amino}-2-oxoethyl)tetrahydro-2H-thiopyran-4-carboxamide 1,1-dioxide,
   N-(4-methylphenyl)-N-(2-{[4-(1,3-oxazol-4-yl)phenyl]amino}-2-oxoethyl)tetrahydro-2H-thiopyran-4-carboxamide 1,1-dioxide,
   N-(3-methylphenyl)-N-(2-{[4-(1,3-oxazol-4-yl)phenyl]amino}-2-oxoethyl)tetrahydro-2H-thiopyran-4-carboxamide 1,1-dioxide,
   N-(2-methylphenyl)-N-(2-{[4-(1,3-oxazol-4-yl)phenyl]amino}-2-oxoethyl)tetrahydro-2H-thiopyran-4-carboxamide 1,1-dioxide,
   N-(2,4-dimethylphenyl)-N-(2-{[4-(1,3-oxazol-4-yl)phenyl]amino}-2-oxoethyl)tetrahydro-2H-thiopyran-4-carboxamide 1,1-dioxide,
   N-(3,4-dimethylphenyl)-N-(2-{[4-(1,3-oxazol-4-yl)phenyl]amino}-2-oxoethyl)tetrahydro-2H-thiopyran-4-carboxamide 1,1-dioxide,
   N-(2,3-dihydro-1H-inden-5-yl)-N-(2-{[4-(1,3-oxazol-4-yl)phenyl]amino}-2-oxoethyl)tetrahydro-2H-thiopyran-4-carboxamide 1,1-dioxide,
   N-(4-chloro-3-methylphenyl)-N-(2-{[4-(1,3-oxazol-4-yl)phenyl]amino}-2-oxoethyl)tetrahydro-2H-thiopyran-4-carboxamide 1,1-dioxide,
   N-(3-fluoro-4-methylphenyl)-N-(2-{[4-(1,3-oxazol-4-yl)phenyl]amino}-2-oxoethyl)tetrahydro-2H-thiopyran-4-carboxamide 1,1-dioxide,
   N-(3-fluoro-2,4-dimethylphenyl)-N-(2-{[4 (1,3-oxazol-4-yl)phenyl]amino}-2-oxoethyl)tetrahydro-2H-thiopyran-4-carboxamide 1,1-dioxide,
   N-(3,5-difluoro-4-methylphenyl)-N-(2-{[4-(1,3-oxazol-4-yl)phenyl]amino}-2-oxoethyl)tetrahydro-2H-thiopyran-4-carboxamide 1,1-dioxide,
   N-(2-fluoro-4-methylphenyl)-N-(2-{[4-(1,3-oxazol-4-yl)phenyl]amino}-2-oxoethyl)tetrahydro-2H-thiopyran-4-carboxamide 1,1-dioxide,
   N-(2,3-dimethylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(2,4-dimethylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(2,6-dimethylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(4-fluoro-2,6-dimethylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(2,3-dihydro-1H-inden-5-yl)-N-(2-{[4-(1,2,4-oxadiazol-3-yl)phenyl]amino}-2-oxoethyl)tetrahydro-2H-thiopyran-4-carboxamide 1,1-dioxide,
   N-(3-fluoro-4-methylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(4-chloro-3-methylphenyl)-N-(2-{[4-(1,2,4-oxadiazol-3-yl)phenyl]amino}-2-oxoethyl)tetrahydro-2H-thiopyran-4-carboxamide 1,1-dioxide, and
   N-(3-fluoro-2,4-dimethylphenyl)-N-(2-{[4-(1,2,4-oxadiazol-3-yl)phenyl]amino}-2-oxoethyl)tetrahydro-2H-thiopyran-4-carboxamide 1,1-dioxide.

In addition, the invention also relates to a use of the N-{2-[(4-substituted phenyl)amino]-2-oxoethyl}tetrahydro-2H-thiopyran-4-carboxamide compound represented by the aforementioned general formula (I) for the manufacture of a medicament for prevention or treatment of diseases caused by acyclovir-resistant herpesviruses.

According to the invention, the ACV-resistant herpesviruses are herpesviruses having resistance to acyclovir, preferably ACV-resistant varicella-zoster virus (VZV) and ACV-resistant herpes simplex virus type 1 and -2 (HSV-1 and HSV-2).

### Effect of the Invention

The compound of the present invention has an excellent anti-viral activity against ACV-resistant herpesviruses and is useful as anti-ACV-resistant herpesvirus agents for the prevention or treatment of various ACV-resistant herpesvirus infections such as varicella (chickenpox) and herpes zoster, caused by ACV-resistant VZV infection, and labial herpes, herpes encephalitis and genital herpes associated with ACV-resistant HSV-1 and ACV-resistant HSV-2 infections.
In addition, the compound of the invention has excellent pharmacokinetics in comparison with the conventional anti-herpesvirus agents, and shows excellent antiviral activity even by a low dose oral administration. Also, different from the nucleic acid-based medicaments, it has low possibility of showing mutagenicity and therefore has high safety.

### Best Mode for Carrying Out the Invention

The active ingredient of the invention, an N-{2-[(4-substituted phenyl)amino]-2-oxoethyl}tetrahydro-2H-thiopyran-4-carboxamide compound of the general formula (I), is further described.
In the invention, F, Cl, Br and I atoms can be exemplified as a "halogen atom".
The N-{2-[(4-substituted phenyl)amino]-2-oxoethyl}tetrahydro-2H-thiopyran-4-carboxamide compound of the invention represented by the general formula (I) also includes its hydrates, various solvates and polymorphic substances.

The following describes typical production methods of the compound (I) which is the active ingredient of the invention. In this connection, the production methods are not limited to the following examples.
In the following production methods, it is sometimes effective from the viewpoint of the production technique to replace a certain functional group depending on the type with an appropriate protective group, namely a group readily convertible to the functional group, at the stage of a raw material or intermediate. Afterwards, the protective group can be eliminated, if necessary, to obtain the desired compound. Examples of such a functional group includes an amino group, hydroxyl group, carboxyl group and the like. Protective groups thereof are, for example, those described in Protective Groups in Organic Synthesis, the third edition (T. W. Green and P. G. M. Wuts, eds., JOHN WILLY & SONS, INC.). These may be appropriately used depending on the reaction conditions. For introducing and eliminating such protective groups, the methods described in the reference can be suitably applied.

### First Production Method

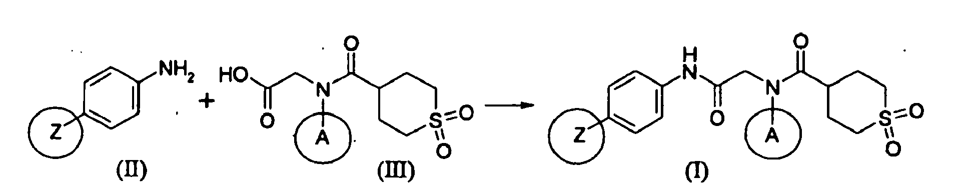

Compound (I) can be easily produced by subjecting Carboxylic Acid Compound (III) and Aniline Derivative (II) to an amidation reaction.
The amidation reaction can be carried out by general methods. For example, the method described in "Courses in Experimental Chemistry" edited by the Chemical Society of Japan, the fourth edition (Maruzen), Vol.22, pp.137-173 may be applicable. Preferably, the reaction is carried out by converting Carboxylic Acid Compound (III) to a reactive derivative such as an acid halide (acid chloride, etc.) or an acid anhydride, and then reacting the resulting reactive derivative with Aniline Derivative (II). In the case of using a reactive derivative of carboxylic acid, a base [an inorganic base such as potassium carbonate, sodium hydroxide, etc. or an organic base such as triethylamine (TEA), diisopropylethylamine, pyridine, etc.] is preferably added. In addition, the amidation reaction may be carried out by reacting carboxylic acid in the presence of a condensation agent [1-ethyl-3-(3-dimethylaminopropyl)carbodiimide (WSC), 1,1'-carbonylbis-1H-imidazole (CDI), etc.]. In this case, additives such as 1-hydroxybenzotriazole (HOBt), etc. may be added. The reaction temperature can be appropriately selected depending on the raw material compound used. The solvent usable includes those inert to the reaction, for example, aromatic hydrocarbon-series solvents such as benzene, toluene, etc.; ether-series solvents such as tetrahydrofuran (THF), 1,4-dioxane, etc.; halogenated hydrocarbon-series solvents such as dichloromethane, chloroform, etc.; amide-series solvents such as N,N-dimethylformamide (DMF), N,N-dimethylacetamide, etc.; basic solvents such as pyridine, etc. The solvent is appropriately selected depending on the type of the raw material compound, and can be used alone or as a mixture of two or more of them.

The aforementioned raw material compounds can be easily produced using known reactions, e.g., those described in "Courses in Experimental Chemistry" edited by the Chemical Society of Japan (Maruzen), in the pamphlet of the International Publication WO 02/38554. The typical production methods thereof are described below.

### Production method of Compound (III)

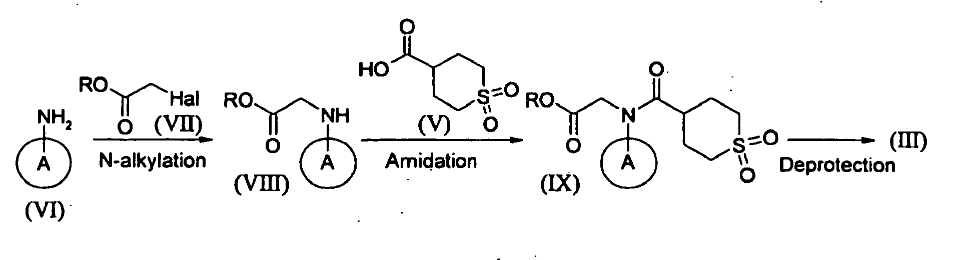

(In the formula, Hal means halogen, R means a group capable of forming an ester residue, such as a lower alkyl, an aralkyl, etc.)
In the reaction scheme above, amidation can be carried out in the same manner as in the first production method above.
N-alkylation of Compound (VI) can be carried out using Halogenated Alkyl Compound (VII) according to usual methods, e.g., the method described in the aforementioned "Courses in Experimental Chemistry", the fourth edition (Maruzen), Vol.20, pp.279-318. The reaction can be carried out under the temperature of from cooling to heating. Examples of the solvent usable include solvents inert to the reaction, for example, those exemplified for the amidation in the first production method, etc. The reaction is carried out preferably in the presence of a base such as potassium carbonate, sodium hydroxide, sodium hydride, etc. The amidation can be carried out in the same manner as in the first production method above. Herein, the amidation may be first carried out and subsequently, the N-alkylation may be carried out.

Deprotection for obtaining Carboxylic Acid Compound (III) can be carried out by appropriately applying a general method depending on the ester type. In the case of alkyl esters such as an ethyl ester, etc., the deprotection can be preferably carried out by treating them with a base such as sodium hydroxide aqueous solution, etc. In the case of aralkyl esters such as a benzyl ester, etc., the deprotection can be carried out by reducing them with palladium-carbon (Pd-C) under hydrogen atmosphere. The reactions can be carried out according to the method described in the aforementioned "Protective Groups in Organic Synthesis", the third edition.
A desired raw material compound can be produced by subjecting the compound with a certain substituent type to a substituent modification reaction well known to those skilled in the art.
The compound (I) of the present invention obtained in this manner is isolated and purified in its free form or as a salt thereof after a salt formation process by a general method. The isolation and purification are carried out by employing general chemical procedures such as extraction, concentration, evaporation, crystallization, filtration, recrystallization, various chromatographic techniques.

The pharmaceutical composition of the present invention, which contains as effective components one type or two or more types of the compound (I) of the present invention, can be prepared according to a method usually used by using pharmaceutical carriers, excipients and the like for general use in this field. Administration thereof may be either oral via tablets, pills, capsules, granules, powders, liquids, etc. or parenteral dosing via injections such as intravenous injections, intramuscular injections, etc., external agents such as ointments, plasters, creams, jellies, cataplasm, sprays, lotions, eye drops, eye ointments, etc., suppositories, inhalation agents.

As the solid composition for oral administration, tablets, powders, granules and the like are used. In such a solid composition, one or more active substances are mixed with at least one inert excipient, for example, lactose, mannitol, glucose, hydroxypropyl cellulose, microcrystalline cellulose, starch, polyvinylpyrrolidone, magnesium metasilicate aluminate, etc. According to general methods, the composition may contain inert additives such as lubricants, e.g., magnesium stearate, etc.; disintegrators, e.g., sodium carboxymethyl starch, etc.; and dissolution auxiliary agents. The tablets or pills may be coated with sugar coating or stomach-soluble or enteric coating.
Examples of the liquid composition for oral administration include pharmaceutically acceptable emulsions, liquids, suspensions, syrups, elixirs, etc., in which inert solvents for general use such as purified water, ethanol, etc. can be incorporated. In addition to the inert solvents, the composition may further contain auxiliary agents such as solubilizing agents, moistening agents and suspending agents; sweetening agents; flavoring agents; aromatic agents and preservatives.
Examples of the injections for parenteral administration include sterile aqueous or non-aqueous liquids, suspensions and emulsions. The aqueous solvents include, for example, distilled water for injections and physiological saline. The non-aqueous solvents include, for example, propylene glycol, polyethylene glycol, vegetable oils such as olive oil, alcohols such as ethanol, Polysorbate 80 (name in the Pharmacopeia). Such compositions may further contain isotonic agents, antiseptics, moistening agents, emulsifying agents, dispersing agents, stabilizers and dissolution auxiliary agents. These are sterilized by filtering through bacteria-retaining filters, by incorporating sterilizing agents, or by irradiation. Alternatively, these may be produced into a sterile solid composition and then dissolved or suspended in sterile water or sterile solvents for injections prior to use.

Examples of the external agents include ointments, plasters, creams, jellies, cataplasms, sprays, lotions, eye drops, eye ointments. The external agent contains generally used ointment bases, lotion bases, aqueous or non-aqueous liquids, suspensions, emulsions.
As the ointment or lotion bases, polyethylene glycol, propylene glycol, white Vaseline, white beeswax, polyoxyethylene hardened castor oil, glycerin monostearate, stearyl alcohol, cetyl alcohol, lauromacrogol, sorbitan sesquioleate, carboxyvinyl polymer, can be mentioned as examples.

Generally, the suitable daily dose of the compound (I) of the present invention, which is the active ingredient of the present invention, is about 0.001 to 50 mg/kg/body weight, preferably 0.01 to 30 mg/kg/body weight, more preferably 0.05 to 10 mg/kg/body weight, for oral administration. For intravenous administration, the daily dose is about 0.0001 to 10 mg/kg/body weight, preferably 0.001 to 1.0 mg/kg/body weight. The dose is administered once or in separate portions per day, and is appropriately determined depending on each case, in terms of the symptom, age, sex. When the compound (I) is to be used as an external agent, the agent containing the compound of the invention in an amount of 0.0001 to 20%, preferably 0.01 to 10%, is desirable. The external agent is administered locally once or in separate portions per day depending on the symptom.

### Examples

Effects of the preventive or therapeutic agent of the invention for diseases caused by ACV-resistant herpesviruses were confirmed by the pharmacological tests shown in the following examples.

### Example 1 Anti-ACV-resistant VZV activity assay

This assay was carried out in reference to the method described by Shigeta S. in The Journal of Infectious Diseases, 147, 3, 576 - 584, (1983). Specifically, 10,000 cells of human embryonic fibroblast (HEF) were inoculated into a 96 well microtiter plate using a growth medium (Eagle MEM (Nissui) supplemented with 10% (v/v) fetal bovine serum (FBS, Sigma)) and cultured at 37°C for 4 days under 5% CO₂ until they became a monolayer. After washing the cells with a maintenance medium, ACV-resistant VZV (Kanno-Br^{ACV-R} strain) which had been diluted with the maintenance medium (Eagle MEM supplemented with 2% FBS) to a viral titer of from 20 to 70 pfu/100 µl was inoculated therein in 100 µl/well portions. The plate was centrifuged at room temperature at 2000 rpm for 20 minutes and then incubated at 37°C for 3 hours under 5% CO₂ for infection with VZV. After washing three times with the maintenance medium, 100 µl of each test drug diluted to an appropriate concentration with the maintenance medium was added to each well. After culturing the cells at 37°C for 3 to 4 days under 5% CO₂, 10% formalin/PBS was added thereto in 100 µl/well portions, and the cells were fixed for 2 to 3 hours. The fixing liquid and culture supernatant were discarded and the plate was washed with water, and then staining was carried out for 2 to 3 minutes by adding a staining liquid (0.025% Crystal Violet) in 50 µl/well portions, and the plate was washed with water and dried at 37°C. The HEF cells infected with VZV cause cell death, and plaques consisting of the dead cells are formed in the mono-layered HEF cells. By counting the number of plaques under a microscope, EC₅₀ value of each test drug was calculated as a concentration which inhibits 50% of the number of plaques.
EC₅₀ values (µM) of the compounds (I) as the active ingredients of the invention are shown in Table 1 which is described later. These compounds were possessed of excellent antiviral activity against the ACV-resistant VZV.

### Example 2 Anti-ACV-resistant HSV-1 activity assay

A total of from 100,000 to 150,000 cells of an African green monkey kidney cell (Vero cell) were inoculated into a 24 well plate using a growth medium (Eagle MEM (Nissui) supplemented with 10% FBS) and cultured at 37°C for 3 to 4 days under 5% CO₂ until they became a monolayer. After washing the cells with a maintenance medium, ACV-resistant HSV-1 (A4-3^{ACV-R} strain) which had been diluted with the maintenance medium (Eagle MEM supplemented with 2% FBS) to a viral titer of from 100 to 250 pfu/200 µl was inoculated therein in 200 µl/well portions. The plate was centrifuged at room temperature at 1500 rpm for 40 minutes and then incubated at 37°C for 1 hour under 5% CO₂ for infection with HSV-1. After washing three times with the maintenance medium, 100 µl of each test drug diluted to an appropriate concentration with the maintenance medium was added to each well. After culturing the cells at 37°C for 3 to 4 days under 5% CO₂, 10% formalin/PBS was added thereto in 200 µl/well portions, and the cells were fixed for 2 to 3 hours. The fixing liquid and culture supernatant were discarded and the plate was washed with water, and then staining was carried out for 2 water, and then staining was carried out for 2 to 3 minutes by adding a staining liquid (0.025% Crystal Violet) in 100 µl/well portions, and the plate was washed with water and dried at 37°C. The Vero cells infected with HSV-1 cause cell death, and plaques consisting of the dead cells are formed in the mono-layered Vero cells. By counting the number of plaques under a microscope, EC₅₀ value of each test drug was calculated as a concentration which inhibits 50% of the number of plaques.
EC₅₀ values (µM) of the compounds (I) as the active ingredients of the invention are shown in the following Table 1. These compounds were possessed of excellent antiviral activity against the ACV-resistant HSV-1.

**[Table 1]**

| | Compound to be tested | Example 1 | Example 2 |
|---|---|---|---|
| Assay | Compound of Preparation 2 | 0.035 | 0.035 |
| | ACV | 24 | 68 |
| Assay 2 | Compound of Preparation 27 | 0.082 | 0.068 |
| | ACV | 27 | 120 |

### Example 3 Pharmacokinetics in in vivo animal model

Using a cutaneous HSV-1 infection mouse model prepared in reference to the method of H. Machida et al. (Antiviral Res., 1992, 17, 133 - 143), the *in vivo* pharmacokinetics of the compounds of the present invention was tested using an animal model. The skin of each HR-1 hairless mouse [female, 7 weeks of age] was scratched lengthwise and breadthwise several times using a needle and a virus suspension (HSV-1 strain WT-51, 1.5 x 10⁴ PFU/15 µl) was dropped to the scarified region for infection, while anesthetized with diethyl ether.
Tested compounds were administered orally as a methyl cellulose suspension, except for compounds marked with asterisk which were dissolved in 20% Cremophor EL (Nacalai Tesque) / 20% polyethylene glycol (PEG) 400 / 60% H₂O solution, starting at 3 hours after the infection, and then at a dose of 10 mg/kg twice a day for 5 days. The symptom of the skin lesion caused by HSV-1 infection were classified in the following scores for 17 days:
Score 0: no signs of infection.
Score 1: localized, barely perceptible small vesicles.
Score 2: slight vesicle spread.
Score 3: large patches of vesicles formed.
Score 4: zosteriform vesicles.
Score 5: large patches of ulcers formed.
Score 6: zosteriform with severe large ulcers.
Score 7: hind limb paralysis or death.
The AUC value was calculated from each group's mean disease score, and the disease inhibitory rate of the group administered with each test compound to the placebo group was calculated using the AUC. The results are shown in Table below.

**[Table 2]**

| Compound to be tested | Inhibition ratio (%) | Compound to be tested | Inhibition ratio (%) |
|---|---|---|---|
| Preparation 1 | * 93 | Preparation 19 | 71 |
| Preparation 2 | 85 | Preparation 20 | 91 |
| Preparation 3 | 70 | Preparation 24 | 89 |
| Preparation 4 | 77 | Preparation 25 | * 70 |
| Preparation 6 | 92 | Preparation 27 | 86 |
| Preparation 8 | 91 | Preparation 31 | * 86 |
| Preparation 11 | 92 | Preparation 33 | 79 |
| Preparation 13 | 89 | Preparation 35 | 82 |
| Preparation 14 | 98 | Preparation 37 | 100 |
| Preparation 17 | 94 | Preparation 39 | 80 |
| Comparative compound A | 38 | Comparative compound B | 2 |
| Comparative compound C | 44 | Comparative compound D | 43 |

### Comparative Compound A:

### Compound of Example 49, Patent Reference 1

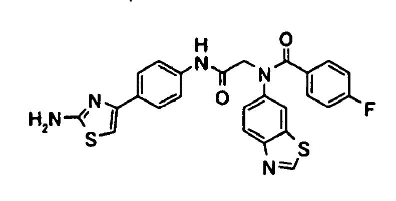

### Comparative Compound B:

### Compound of Example 85, Patent Reference 1

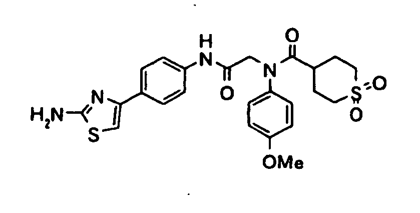

### Comparative Compound C:

### Compound of Example 87, Patent Reference 1

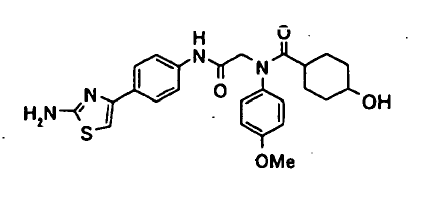

### Comparative Compound D:

### Compound of Example 119, Patent Reference 1

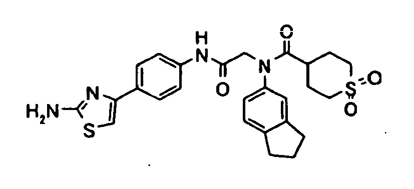

In the group in which the compounds (I) as the active ingredients of the invention were administered, it was confirmed that worsening of the symptom in the lesion moiety caused by the HSV-1 infection was properly controlled by reflecting their *in vitro* activities, and the activities were superior to those of the analogous compounds described in Patent Reference 1. Accordingly, it was confirmed that the compounds (I) as the active ingredients of the invention have excellent pharmacokinetics in the in vivo animal model.

### Example 4 Evaluation of effect using an ACV-resistant HSV-1 infection lethal model in nude mice

ACV-resistant HSV-1 (3 x 10⁶ pfu) was inoculated into the peritoneal cavity of each nude mouse (CD-1(ICR)-nu/nu, 5 weeks of age, female, Charles River Japan) to cause infection with the virus. Each compound to be tested was made into a methyl cellulose suspension and orally administered at a dose of 100 mg/kg, twice a day for 15 days starting 1 hour after the infection (from 0 to 14 days after the infection).
During 17 days after the infection, death of the mice caused by ACV-resistant HSV-1 infection was observed every day, and the number of survived animals was counted. The results are shown in the following table. Though clear death suppressing effect was not found by VCV as the control drug, the compounds of the Preparation 2 and 27 of the instant application completely suppressed death of the mice.
Accordingly, it was confirmed that the compounds of the invention properly suppress infections with ACV-resistant herpesviruses also in the *in vivo* animal model.

**[Table 3]**

| | The number of survived animals (n = 10) | | | | | | | | | | | | | | |
|---|---|---|---|---|---|---|---|---|---|---|---|---|---|---|---|
| Days after infection | 0 | 1 | 2 | 4 | 5 | 6 | 6 | 8 | 9 | 10 | 11 | 12 | 14 | 16 | 17 |
| Control (no viral infection) | 10 | 10 | 10 | 10 | 10 | 10 | 10 | 10 | 10 | 10 | 10 | 10 | 10 | 10 | 10 |
| Control 2 (viral infection) | 10 | 10 | 10 | 10 | 10 | 10 | 10 | 7 | 2 | 1 | 1 | 1 | 1 | 1 | 1 |
| ompound of Preparation 2 | 10 | 10 | 10 | 10 | 10 | 10 | 10 | 10 | 10 | 10 | 10 | 10 | 10 | 10 | 10 |
| Compound of Preparation 27 | 10 | 10 | 10 | 10 | 10 | 10 | 10 | 10 | 10 | 10 | 10 | 10 | 10 | 10 | 10 |
| Comparative example (VCV) | 10 | 10 | 10 | 10 | 10 | 10 | 9 | 8 | 4 | 2 | 2 | 2 | 2 | 2 | 2 |

### (Preparations)

Production Examples of the compound (I), which is an active ingredient of the present invention, are shown below. Herein, many of the raw material compounds for use in the following reactions are known in the pamphlet of the Patent Reference 1 (International Publication WO 02/38554) and the like, and can therefore be readily available according to the methods described in these known references. Production examples of novel compounds among the raw materials are shown below in Reference Examples.

### Reference Example 1:

5% Palladium-carbon powder was added to an ethanol-tetrahydrofuran mixed suspension of 4-(4-nitrophenyl)-1,3-oxazol and stirred for 12 hours at room temperature in a hydrogen atmosphere. The reaction solution was filtered through Celite and the filtrate was evaporated under reduced pressure. The resulting crude product is purified with a silica gel column chromatography to obtain [4-(1,3-oxazol-4-yl)phenyl]amine (pale yellow solid). Electron Impact-MS(M)⁺: 160.

### Reference Example 2:

Potassium carboxylate and ethyl bromoacetate were added to a DMF solution of 4-methylaniline and heated while stirring. The reaction mixture was added with water and ethyl acetate. After the organic layer was separated, washed and dried, the solvent was evaporated under reduced pressure to obtain a crude product. The crude product was dissolved in methylene chloride, and pyridine, tetrahydro-2H-thiopyrane-4-carbonyl chloride 1,1-dioxide were added to the resulting solution and stirred. After the reaction solution was concentrated, 1M hydrochloric acid and chloroform were added. The organic layer separated was washed and dried and the solvent was evaporated under reduced pressure. The resulting crude product was purified with a silica gel column chromatography to obtain ethyl {[(1,1-dioxotetrahydro-2H-thiopyran-4-yl)carbonyl](4-methylphenyl)amino}acetate (colorless oily product). FAB-MS [(M+H)⁺]: 354.

### Reference Examples 3 to 15:

Compounds of Reference Examples 3 to 15, which are described in Table 4 below, were obtained in the same manner as in Reference Example 2.

### Preparation 1:

To an ethanol (10 ml) solution of ethyl {(2,6-dimethylphenyl)[(1,1-dioxide tetrahydro-2H-thiopyran-4-yl)carbonyl]amino}acetate (735 mg) was added aqueous 1M sodium hydroxide solution (2.3 mL). The mixture was stirred at room temperature for 5 hours. After 1M hydrochloric acid was added to the reaction mixture to make the solution acidic, water and chloroform were added thereto to separate the organic layer. Further, the organic layer was dried over anhydrous sodium sulfate and filtered, and then, the solvent was evaporated under reduced pressure. After the resulting crude carboxylic acid product was dissolved in chloroform (15 ml), WSC·HCl (422 mg) and [4-(1,3-oxazol-4-yl)phenyl]amine (320 mg) were added sequentially to the resulting solution, which was stirred at room temperature for 4 hours. After a saturated sodium hydrogencarbonate aqueous solution and chloroform were added to the reaction solution, the organic layer was separated. The organic layer was washed with a saturated sodium chloride solution, dried over anhydrous magnesium sulfate and filtered, from which the solvent was evaporated under reduced pressure. The resulting crude product was rinsed in hexane-ethyl acetate (= 3/2), and then recrystallized from ethanol, to obtain N-(2,6-dimethylphenyl)-N-(2-{[4-(1,3-oxazol-4-yl)phenyl]amino}-2-oxoethyl)tetrahydro-2H-thiopyran-4-carboxamide 1,1-dioxide (colorless crystal) in a yield of 610 mg.

### Preparations 2 - 40:

Compounds of Preparations 2 to 40 shown in Tables 5 to 12 below were obtained in the same manner as in Preparation 1.
The physicochemical properties of the compounds of Reference Examples are shown in Table 4, while Tables 5 to 12 show the structures and physicochemical properties of the compounds of Preparations.

Abbreviations in the tables indicate as follows.
Ref: Reference Example; Ex: Preparation; Dat: physicochemical properties {F+: FAB-MS [(M+H)⁺].; F-: FAB-MS [(M-H)⁻]; N1: ¹H-NMR (DMSO-d₆, TMS internal standard); mp: melting point (°C), solvent for crystallization is shown in the parentheses}; Ph: phenyl; Me: methyl; Et: ethyl; and iPr: isopropyl. Herein, the numerical figure before each substituent indicates the position for its substitution. For example, 3,4-(Cl)₂-5-F-Ph indicates a 3,4-dichloro-5-fluorophenyl group.

**[Table 4]**

| | | | | | | | |
|---|---|---|---|---|---|---|---|
| 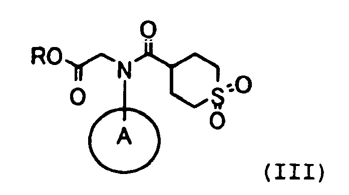 | | | | | | | |

| Ref | A | R | Dat | Ref | A | R | Dat |
|---|---|---|---|---|---|---|---|
| 3 | 2,3-(Me)₂-Ph | Et | F+:368 | 4 | 4-M+Ph | Et | F+:354 |
| 5 | 2,5-(Me)₂-Ph | Et | F+:368 | 6 | 3-Me-Ph | Et | F+:354 |
| 7 | 3.4-(Me)₂-Ph | Et | F+:368 | 8 | 2-Me-Ph | Et | F+:354 |
| 9 | 2,4,6-(Me)₃-Ph | Et | F+: 382 | 10 | 2,4-(Me)₂-Ph | Et | F+: 368 |
| 11 | 4-F-3-Me-Ph | Et | F+:372 | 12 | 2,6-(Me)₂-Ph | Et | F+:368 |
| 13 | 3-Br-4-Me-Ph | Et | F+: 432, 434 | 14 | 4-F-2,6-(Me)₂-Ph | Et | F+:386 |
| 15 | 3,5-(Me)₂-Ph | Et | F+:368 | | | | |

**[Table 5]**

| | | |
|---|---|---|
| 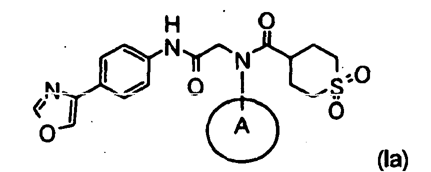 | | |

| Ex | A | Dat |
|---|---|---|
| | | F+:482 |
| 1 | 2,6-(Me)₂-Ph | N1: 1.87-2.42(5H, m), 2.13(6HX0.1, s), 2.33(6HX0.9, s), 2.97-327(4 H, m), 4.19(2HX0.9, s), 4.48(2HX0.1, s), 7.07-7.25(3H, m), 7.62-7 .66(2H, m), 7.72-7.75(2H, m), 8.43(1H, d), 8.54(1H, d), 10.15(1H, brs) |
| | | mp: 224-227 (EtOH) |
| | | F+: 468 |
| 2 | 4-Me-Ph | N1: 1.98-2.06(4H, m), 2.34(3H, s), 2.68-2.70(1H, m), 2.97-3.02(4H, m), 4.35(2H, s), 7.28 (2H, d), 7.36(2H, d), 7.63-7.66(2H. m), 7.7 2-7.76(2H, m), 8.43(1H, s), 8.54(1H, s), 10.14(1H, s) |
| | | mp: 199-201 (EtOHHH₂O) |
| | | F+: 468 |
| 3 | 3-Me-Ph | N1: 2.01-2.09(4H, m), 2.35(3H, s), 2.71(1H, m), 2.93-3.06(4H, m), 4 .36(2H, s), 7.17-7.38 (4H, m), 7.64(2H, d), 7.73(2H, d), 8.43(1H, d), 8.54(1H, d), 10.15(1H, s) |
| | | F+: 468 |
| 4 | 2-Me-Ph | N1: 1.88-2.15(4H, m), 2.15(3HX0.1, s), 2.26(3HX0.9, s), 2.41-2.46(1 H, m), 2.83-3.05(4H, m), 3.86(1HX0.9, d), 4.20(1HX0.1, d), 4.74( 1HX0.9, d), 4.84(1HX0.1, d), 7.09-7.77(8H, m), 8.43(1H, d), 8.53( 1H, d), 10.14(1HX0.9, s), 10.19(1HX0.1, s) |
| | | mp: 220-221 (EtOH) |

**[Table 6]**

| | | |
|---|---|---|
| | | F+: 482 |
| 5 | 2,3-(Me)₂-Ph | N1: 1.85-2.12(4H, m), 2.03(3HX0.1, s), 2.15(3HX0.9, s), 2.25(3HX0. 1, s), 2.31(3HX0.9, s), 2.42-2.47(1H, m), 2.83-2.90(1H, m), 3.00-3 .22(3H, m), 3.84(1HX0.9, d), 4.16(1HX0.1, d), 4.72(1HX0.9, d), 4. 84(1HX0.1, d), 7.07-7.36(3H, m), 7.62-7.66(2H. m), 7.71-776(2H, m), 8.43(1H, brs), 8.54(1H, d), 10.12(1HX0.9, s), 10.16(1HX0.1, s) |
| | | mp: 185-187 (EtCH) |
| | | F+: 482 |
| 6 | 2,4-(Me)₂-Ph | N1: 1.88-2.50(5H, m), 2.09(3HX0.1, s), 2.21(3HX0.9, s), 2.25(3HX0. 1, s), 2.30(3HX0.9. s), 2.85-3.20(4H, m), 3.81(1HX0.9, d). 4.17(1 HX0.1, d), 4.72(1HX0.9, d), 4.81(1HX0.1, d), 6.97-7.39(3H, m), 7. 62-7.66(2H, m), 7.72-7.76(2H, m), 8.43(1H, s), 8.54(1H, s), 10.11 (1HX0.9, s), 10.17(1HX0.1. s) |
| | | mp: 176-177 (EtCH) |
| | | F+: 482 |
| 7 | 2,5-(Me)₂-Ph | N1: 1.86-2.51 (5H. m), 2.08(3HX0.1, s), 2.20(3HX0.9, s), 2.22(3HX0. 1, s), 2.30(3HX0.9. s), 2.87-3.26(4H, m), 3.84(1HX0.9, d), 4.21(1 HX0.1, d), 4.70(1HX0.9, d), 4.80(1HX0.1, d), 6.92-70.32(3H, m) 7. 63-7.65(2H, m), 7.72-7.76(2H, m), 8.43(1H, s), 8.54(1H, s), 10.12 (1HX0.9, s), 10.17(1HX0.1, s) |
| | | mp: 201-202 (EtCH) |
| | | F+- 482 |
| 8 | 3,4-(Me)₂-Ph | N1: 1.92-2.08(4H, m), 2.09(3H, s), 2.24(3H, s), 2.71 (1H, s), 2.94-3. 06(4H, m), 4.33(2H, S), 7.17-7.24(3H, m), 7.64(2H, d), 7.73(2H, d), 8.43(1H, s), 8.54(1H, s), 10.12(1H, s) |
| | | F+: 482 |
| 9 | 3,5-(Me)₂-Ph | N1: 1.96-2.14(4H, m), 2.30(6H, s), 2.73(1H, m), 2.95-3.04(4H, m), 4 N1: 33(2H, S), 7.02(1H, s), 708(2H, s), 7.64(2H, d), 773(2H, d), 8.4 3(1H, s), 8.54(1H, s), 10.12(1H, s) |
| | | mp: 205-206 (EtOH) |

**[Table 7]**

| | | |
|---|---|---|
| | | F+: 496 |
| 10 | 2,4,6(Me)₋₃Ph | N1: 1.87-2.45(5H, m), 2.08(3HX0.1, s), 2.09(6HX0.1, s), 2:27(3HX0. 9, s), 2.28(6HX0.9, s), 3.01-3.26(4H, m), 4.16(2HX0.9, s), 4.44(2 HX0.1, s), 6.88(2HX0.1, s), 7.01(2HX0.9, s), 7.61-7.65(2H, m), 7. 71-7.75(2H, m), 8.43(1H, s), 8.54(1H, s), 10.12(1HX0.9, s), 10.14 (1HX0.1, s) |
| | | mp: 237-238 (EtCH) |
| | | F+: 494 |
| 11 | 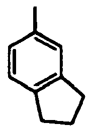 | N1: 2.01-2.08(6H, m), 2.70-3.06(9H, m), 4.34(2H, s), 7.13-7.32(3H, m), 7.64(2H, d), 7.73(2H, d), 8.43(1H, s), 8.54(1H, s), 10.13(1H, s) |
| | | F+: 502 |
| 12 | 3-Cl-4-Me-Ph | N1: 2.01-2.06(4H, m), 2.36(3H, s), 2.68-2.75(1H, m), 3.01-3.06(4H, m), 4.37(2H, S), 7.37-7.40(1H, m), 7.46(1H, d), 7.60-7.66(3H, m), 7.74(2H, d), 8.44(1H, s), 8.55(1H, s), 10.18(1H, s) |
| | | mp: 146-148 (EtCH) |
| | | F+: 502 |
| 13 | 4-Cl-3-Me-Ph | N1: 2.00-2.06(4H, m), 2.36(3H, s), 2.68.2.75(1H, m), 3.01-3.04(4H, m), 4.36(2H, S), 7.33-7.36(1H, m), 7.48-7.52(2H, m), 7.64(2H, d), 7.73(2H, d), 8.43(1H, s), 8.54(1H, s), 10.18(1H, s) |
| | | mp: 133-135 (EtCH) |
| | | F+: 486 |
| 14 | 3-F-4-Me-Ph | N1: 2.00-2.05(4H, m), 2.26(3H, s), 2.70-2.77(1H, m), 3.01-3.03(4H, m), 4.36(2H, S), 7.24-7.26(1H, m), 7.32-7.41(2H, m), 7.64(2H, d), 7.73(2H, d), 8.43(1H, s), 8.54(1H, s), 10.17(1H, s) |
| | | mp: 135-137 (EtCH) |
| | | F+: 546, 548 |
| 15 | 3-Br-4-Me-Ph | N1: 2.00-2.06(4H, m), 2.38(3H, s), 2.68-2.74(1H, m), 3.01-3.04(4H, m), 4.36(2H, S), 7.41-7.47(2H, m), 7.64(2H, d), 7.73-7.76(3H, d), 8.43(1H, s), 8.54(1H, s), 10.18(1H, s) |
| | | mp: 154-155 (EtCH) |

**[Table 8]**

| | | |
|---|---|---|
| | | F+: 486 |
| 16 | 5-F-2-Me-Ph | N1: 1.88-2.15(4H, m), 2.11(3HX0.1, s), 2.23(3HX0.9, s), 2.45-2.49(1 H, m), 2.96-3.16(4H, m), 3.92(1HX0.9, d), 4.27(1HX0.1, d), 4.70( 1HX0.9, d), 4.82(1HX0.1, d), 6.95-6.98(1HX0.1, m), 7.06-7.10(1H X0.1, m), 7.20-7.25(1HX0.9, m), 7.29-7.33(1HX0.1, m), 7.37-7.7.4 0(1HX0.9, m), 7.42-7.46(1HX0.9, m), 7.65(2H, d), 7.74(2H, d), 8. 43(1H, s), 8.54(1H, s), 10.18(1HX0.9, s), 10.23(1HX0.1, s) |
| | | mp: 235-236 (EtCH) |
| | | F+: 500 |
| 17 | 3-F-2,4-(Me)₂-Ph | N1: 1.88-2.23(4H, m), 2.03(3HX0.1, s), 2.16(3HX0.9, s), 2.20(3HX0. 1, s), 2.26(3HX0.9, s), 2.47-2.54(1H, m), 2.87-3.17(4H, m), 3.91(1 HX0.9, d), 4.25(1HX0.1, d), 4.66(1HX0.9, d), 4.80(1HX0.1, d), 6.8 8 (1HX0.1, d), 7.10(1HX0.1, dd), 7.21 (1HX0.9, dd), 7.28(1HX0.9. d), 7.64(2H, d), 7.73(2H, s), 8.43(1H, s), 8.54(1H, s), 10.14(1HX 0.9, s), 10.20(1HX0.1, s) |
| | | mp: 185-186 (EtOH) |
| | | F+: 500 |
| 18 | 4-F-35(Me)₂-Ph | N1: 2_{.}00-2.05(4H, m), 224(6H, s), 2.67-274(1H, m), 3.00-3.04(4H, m), 4.33(2H, S), 7.23(2H, d), 7.65 (2H, d), 7.74(2H, d), 8.43(1H, s), 8.54(1H, s), 10.15(1H, s) |
| | | mp: 188-189 (EtOH) |
| | | F+: 504 |
| 19 | 3,5-F₂-4-Me-Ph | N1: 1.99-2.05(4H, m), 2.17(3H, s), 2.75-2.82(1H, m), 2.99-3.10(4H, m), 4.37(2H, S), 7.28(2H, d), 7.65 (2H, d), 7.74(2H, d), 8.44(1H, s), 8.55(1H, s), 10.21(1H, s) |
| | | mp: 221-223 (EtCH) |
| | | F+: 486 |
| 20 | 2-F-4-Me-Ph | N1: 1.89-2.11(4H, m), 2.30(3HX0.1, s), 2.36(3HX0.9, s), 2.60-2.68(1 H, m), 3.01-3.26(4H, m), 3.94(1HX0.9, d), 4.02(1HX0.1, d), 4.50( 1HX0.1, d), 4.76(1HX0.9, d), 7.00(1HX0.1, d), 7.09(1HX0.1, d), 7. 12(1HX0.9, d), 7.24(1HX0.9, d), 7.38 (1HX0.1, dd), 7.50(1HX0.9, dd), 7.63(2H, d), 7.73(2H, d), 8.44(1H, s), 8.55(1H, s), 10.17(1HX 0.9, s), 10.23(1HX0.1, s) |

**[Table 9]**

| | | |
|---|---|---|
| 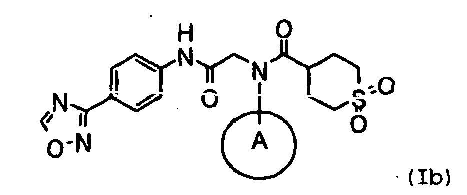 | | |

| Ex | A | Dat |
|---|---|---|
| | | F+:469 |
| 21 | 4-Me-Ph | N1: 1.94-2.11(4H, m), 2.34(3H, s), 2.65-2.75(1H, m), 2.92-3.08(4H, m), 4.38(2H, s), 7.28(2H, d), 7.37(2H, d), 7.79(2H, d), 8.00(2H, d), 9. 66(1H, s), 10.38(1H, s) |
| | | mp: 203-205 (EtOH) |
| | | F-:467 |
| 22 | 3-Me-Ph | N1: 1.96-2.11 (4H, m), 2.35(3H, s), 2.65-2.76(1H, m), 2.92-3.09(4H, m ), 4.39(2H, s), 7.20-7.39(4H, m), 7.79(2H, d), 8.00(2H, d), 9.66(1H, s), 10.38(1H, s) |
| | | F+: 469 |
| 23 | 23 2-Me-Ph | N1: 1.88-2.26(4H+3H, m), 2.42-2.52(1H, m), 2.84-3.18(4H, m), 3.91(1 H × 0.9, d), 4.44(1H × 0.1, d), 4.75(1 H × 0.9, d), 4.87(1H × 0.1, d), 7. 08-7.54(4H, m), 7.75-7.81 (2H, m), 7.97-8.04(2H. m), 9.66(1H × 0.9, s). 9.67(1H×0.1. s), 10.37(1H × 0.9, s), 10.41(1H × 0.1, s) |
| | | mp: 216-217 (MeOH) |
| | | F-: 481 |
| 24 | 2.3-(Me)₂-Ph | N1: 1.83-2.31 (4H+3H+3H, m), 2.42-2.54(1H. m), 2.82-3.16(4H, m), 3. 88(1H × 0.9, d), 4.19(1H × 0.1, d), 4.72(1H × 0.9, d), 4.87(1H × 0.1, d), 7.05-7.37(3H, m), 7.75-7.80(2H, m), 7.97-8.03(2H, m), 9.66(1H × 0.9, s), 9.66(1H × 0.1, s), 10.35(1H × 0.9, s), 10.38(1H × 0.1, s) |
| | | mp: 223-225 (ETOH) |
| | | F-: 481 |
| 25 | 2,4-(Me)₂-Ph | N1: 1.84-2.33(4H+3H+3H, m), 2.42-2.52(1H, m), 2.843.19(4H, m), 3. 86(1H× 0.9, d), 4.21 (1H × 0.1, d), 4.73(1H × 0.9, d), 4.84(1H × 0.1, d), 6.95-7.40(3H, m), 7.75-7.81(2H, m), 7.98-8.02(2H. m), 9.66(1H × 0.9, s), 9.66(1H × 0.1, s), 10.35(1H × 0.9, s), 10.39(1H × 0.1, s) |
| | | mp: 139-141 (EtOH) |

**[Table 10]**

| | | |
|---|---|---|
| | | F-: 481 |
| 26 | 2,5-(Me)₂-Ph | N1: 1.84-2.32(4H+3H+3H, m), 2.42-2.52(1H, m), 2.87-3..18(4H, m), 3. 89(1H×0.9. d), 4.25(1H × 0.1, d), 4.72(1H × 0.9, d), 4.83(1H × 0.1, d), 6.92-7.34(3H, m), 7.76-7.82(2H,m), 7.98-8.04(2H,m), 9,66 (1H m), 9,66(1H × 0.9, s), 9.67(1H × 0.1, s), 10.37(1H × 0.9, s), 10.39(1H × 0.1,s) |
| | | mp: 214-217 (EtOH) |
| | | F-: 481 |
| 27 | 2,6-(Me)₂-Ph - | N1 1.88-2.42(5H+6H, m), 2.98-3.27(4H, m), 422(2H × 0.86, s) 4.51(2 H × 0.14, s), 7.1-7.3(3H, m), 7.76-7.81(2H, m), 7.99-8.03(2H, m), 9. 66(1H, s), 10.38(1H, s) |
| | | mp:220-222(EtOH-H₂O) |
| | | F+: 483 |
| 28 | 3,4-(Me)₂-Ph | N1: 1.97-2.20(4H, m), 224(6H, s), 2.67-2.76(1H, m), 2.96-3.30(4H, m ), 4.37(2H, s), 7.17-7.27(3H, m), 7.79(2H, d), 8.00(2H, d), 9.66(1H, s), 10.36(1H, s) |
| | | mp: 141-143 (EtOH) |
| | | F+:483 |
| 29 | 3,5-(Me)₂-Ph | N1: 1.98-2.12(4H, m), 2.30(6H, s), 2.65-2.78(1H, m), 2.933.10(4H, m ), 4.36(2H, s), 7.00-7.12(3H, m), 7.79(2H, d), 8.00(2H, d), 9.66(1H, s), 10.37(1H; s) |
| | | mp: 197-198 (iPrOH) |
| | | F-: 495 |
| 30 | 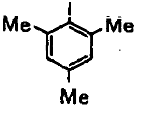 | N1: 1.83-2.52(4H+9H+1H, m), 2.99-3.16(4H, m), 4.18(2H × 0.9, s), 4. 48(2H × 0.1, s), 6.88(2H × 0.1, s), 7.01 (2H × 0.9, s), 7.74-7.82(2H, m), 7.94-8.03(2H, m), 9.66(1H,s), 10.36(1H, s) |
| | | mp: 188-190 (EtOH) |
| | | F-: 499 |
| 31 | 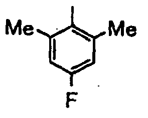 | N1: 1.82-2.44(6H+5H, m), 2.98-3.30(4H, m), 4.21(2H×0.85, s), 4.50( 2H × 0.15, s), 6.95(2H × 0.15, d), 7.08(2H × 0.85, d), 7.75-7.82(2H, m). 7.97-8.04(2H, m), 9.66(1H × 0.85, s), 9.66(1H × 0.15, s), 10.40( 1H, brs) |
| | | mp: 226-229 (EtOH-MeCN-H₂O) |

**[Table 11]**

| | | |
|---|---|---|
| | | F+:487 |
| 32 | 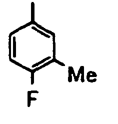 | N1: 1.97-2.11 (4H, m), 2.26(3H, brs), 2.63-2.74(1H, m), 2.95-3.07(4 H, m), 4.38(2H, s), 721-7.45(3H, m), 7.79(2H, d), 8.00(2H, d), 9. 66(1H, s), 10.39(1H, s) |
| | | mp: 136-138 (EtOH) |
| | | F-: 493 |
| 33 | 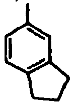 | N1: 1.96-2.20(6H, m), 2.70-2.78(1H, m), 2.84-3.08(8H, m), 4.37(2H, s), 7.04-7.33(3H, m), 7.79(2H, d), 8.00(2H, d), 9.66(1H, s), 10.37 (1H, s) |
| | | F-: 546 |
| 34 | 4-Me-3-Br-Ph | N1: 1.96-2.16(4H, m), 2.38(3H, s), 2.66-2.77(1H, m), 2.96-3.08(4H, m), 4.39(2H, s), 7.40-7.49(2H, m), 7.73-7.82(3H, m), 8.00(2H, d), 9.66(1H, s), 10.41(1H, s) |
| | | mp: 203-206 (iPrOH) |
| | | F+: 487 |
| 35 | 3-F-4Me-Ph | N1: 1.97-2.07 (4H, m), 2.26 (3H, s), 2.69-2.77 (1H, m), 2.99-3.03 (4H, m), 4.39 (2H, s), 7.22-7.28 (1H, m), 7.31-7.42 (2H, m), 7.80 (2H, d), 7.99 (2H, d), 9.66 (1H, s), 10.40 (1H, s) |
| | | mp: 200-203 (EtOH) |
| | | F+: 503 |
| 36 | 3-Cl-4-Me-Ph | N1: 1.97-2.11 (4H, m), 2.36 (3H, s), 2.65-2.78 (1H, m), 2.97-3.08 ( 4H, m), 4.39 (2H, s), 7.39 (1H, dd), 7.45 (1H, d), 7.60 (1H, d), 7. 80 (2H, d), 7.93 (2H, d), 9.65 (1H, s), 10.40 (1H, s) |
| | | mp: 204-205 (EtOH) |
| | | F+: 503 |
| 37 | 4-Cl-3-Me-Ph | N1: 1.95-2.09 (4H, m), 2.36 (3H, s), 2.65-2.76 (1H, m), 2.95-3.07 ( 4H, m), 4.39 (2H, s), 7.36 (1H, dd), 7.48 (1H, d), 7.51 (1H, d), 7. 80 (2H, d), 7.99 (2H, d), 9.66 (1H, s), 10.40 (1H, s) |
| | | mp: 196-199 (EtOH) |
| | | F+: 501 |
| 38 | 4-F-3,5-(Me)₂Ph | N1: 1.94-2.12(4H, m), 2.24(6H, s), 2.64-2.74(1H, m), 2.94-3.08(4H, m), 4.35(2H, s), 7.23(2H, d), 7.79(2H, d), 7.99(2H, d), 9.66(1H, s) 10.38(1H, s) |

**[Table 12]**

| | | |
|---|---|---|
| | | F+: 501 |
| 39 | 3-F-2,4-(Me)₂-Ph | N1: 1.84-2.34(4H+3H+3H, m), 2.48-2.55(1H, m), 2.85-322(4H, m), 398(1H×0.9, d), 4.30(1H × 0.1, d), 4.65(1H × 0.9, d), 4.81(1H× 0 1,d), 7.22(1H, t), 7.27(1H, d), 7.78(2H, d), 7.98(2H, d), 9.66(1H, s), 10.37(1H × 0.9, s), 10.51(1H × 0.1, s) |
| | | F+: 487 |
| 40 | 2-F-4-Me-Ph | N1: 1.90-2.18(4H, m), 2.30(3HX0.1, s), 2.36(3HX0.9, s), 2.62-2.68(1 H, m), 3.01-3.23(4H, m), 3.99(1H, d), 4.T1(1H, d), 7.13(1H, d), 7. 25(1H, d), 7.50(1H, dd), 7.77(2H, d), 7.99(2H, d), 9.66(1H, s), 10. 40(1HX0.9, s), 10.45(1HX0.1, s) |
| | | mp: 197-198 (EtOH) |

### Industrial Applicability

The compounds of the present invention have an excellent anti-viral activity against ACV-resistant herpesviruses and are useful as anti-ACV-resistant herpesvirus agents for the prevention or treatment of various ACV-resistant herpesvirus infections such as varicella (chickenpox) and herpes zoster, caused by ACV-resistant VZV infection, and labial herpes, herpes encephalitis and genital herpes caused by ACV-resistant HSV-1 and ACV-resistant HSV-2 infections.
In addition, the compound of the invention has excellent pharmacokinetics in comparison with the conventional anti-herpesvirus agents, and shows excellent antiviral activity even by a low dose oral administration. Also, different from the nucleic acid-based medicaments, it has low possibility of showing mutagenicity and therefore have high safety.

## Claims

1. A medicament for use in the prevention or treatment of diseases caused by acyclovir-resistant herpesviruses, which comprises an N-{2-[(4-substituted phenyl)amino]-2-oxoethyl}tetrahydro-2H-thiopyran-4-carboxamide compound represented by the following general formula (I) as an active ingredient 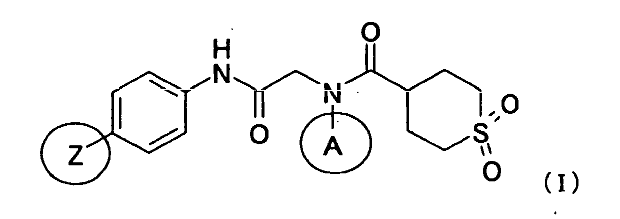 (symbols in the formula represent the following meanings
Z: a 1,2,4-oxadiazol-3-yl or 4-oxazolyl group,
A: a phenyl group which is substituted by at least one methyl group and may further have 1 or 2 substituent groups selected from the group consisting of a methyl group and halogen atoms, or 5-indanyl group).

2. The medicament for use in the prevention or treatment described in claim 1, wherein Z is a 1,2,4-oxadiazol-3-yl group.

3. The medicament for use in the prevention or treatment described in claim 1, wherein Z is a 4-oxazolyl group.

4. The medicament for use in the prevention or treatment described in claim 1, wherein A is a phenyl group which is substituted by at least one methyl group and may further have 1 or 2 substituent groups selected from the group consisting of a methyl group and halogen atoms.

5. Use of the N-{2-[(4-substituted phenyl)amino]-2-oxoethyl}tetrahydro-2H-thiopyran-4-carboxamide compound represented by the formula (I) described in claim 1, for the manufacture of a medicament for prevention or treatment of diseases caused by acyclovir-resistant herpesviruses.

## Patentansprüche

1. Medikament für die Verwendung bei der Verhütung oder Behandlung von durch Acyclovir-resistenten Herpesviren verursachten Erkrankungen, das als Wirkstoff eine N-{2-[(4-substituiertes Phenyl]amino}-2-oxoethyl}tetrahydro-2H-thiopyran-4-carboxamidverbindung enthält, repräsentiert durch die folgende allgemeine Formel (I): 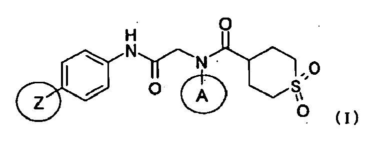 (Symbole in den Formeln haben die folgenden Bedeutungen:
Z: eine 1,2,4-Oxadiazol-3-yl- oder 4-Oxazolyl-Gruppe,
A: eine Phenylgruppe, die durch wenigstens eine Methylgruppe substituiert ist und ferner 1 oder 2 Substitutentengruppen haben kann, ausgewählt aus der Gruppe bestehend aus einer Methylgruppe und Halogenatomen, oder einer 5-Indanylgruppe).

2. Medikament für die Verwendung bei der Verhütung oder Behandlung wie in Anspruch 1 beschrieben, wobei Z eine 1,2,4-Oxadiazol-3-yl-Gruppe ist.

3. Medikament für die Verwendung bei der Verhütung oder Behandlung wie in Anspruch 1 beschrieben, wobei Z eine 4-Oxazolyl-Gruppe ist.

4. Medikament für die Verwendung bei der Verhütung oder Behandlung wie in Anspruch 1 beschrieben, wobei A eine Phenylgruppe ist, die durch wenigstens eine Methylgruppe substituiert ist und ferner 1 oder 2 Substituentengruppen haben kann, ausgewählt aus der Gruppe bestehend aus einer Methylgruppe und Halogenatomen.

5. Verwendung der N-{2-[(4-substituiertes Phenyl]amino}-2-oxoethyl}tetrahydro-2H-thiopyran-4-carboxamidverbindung, repräsentiert durch die in Anspruch 1 beschriebene Formel (I), bei der Herstellung eines Medikaments für die Verwendung bei der Verhütung oder Behandlung von durch Acyclovir-resistenten Herpesviren verursachten Erkrankungen.

## Revendications

1. Médicament destiné à être utilisé dans la prévention ou le traitement de maladies causées par les virus de l'herpès résistants à l'acyclovir, lequel comprend un composé N-(2-[2-[(4-substitué phényl) amino]-2-oxoethyl] tétrahydro-2H-thiopyran-4-carboxamide représenté par la formule générale suivante (I) comme ingrédient actif 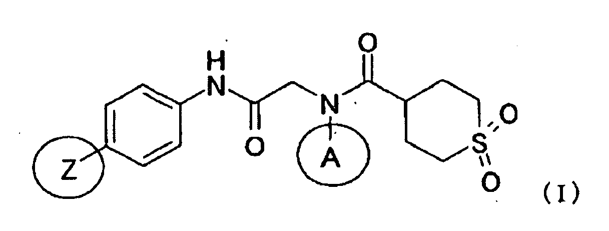 (les symboles de la formule ont les significations suivantes
Z : un groupe 1,2,4-oxadiazol-3-yl ou 4-oxazolyl,
A : un groupe phényle qui est substitué par au moins un groupe méthyle et peut comporter en outre 1 ou 2 groupes substituants sélectionnés dans le groupe consistant en un groupe méthyle et des atomes halogènes, ou un groupe 5-indanyl).

2. Médicament destiné à être utilisé dans la prévention ou le traitement décrit à la revendication 1, dans lequel Z est un groupe 1,2,4-oxadiazol-3-yl.

3. Médicament destiné à être utilisé dans la prévention ou le traitement décrit à la revendication 1, dans lequel Z est un groupe 4-oxazolyl.

4. Médicament destiné à être utilisé dans la prévention ou le traitement décrit à la revendication 1, dans lequel A est un groupe phényl qui est substitué par au moins un groupe méthyle et peut comporter en outre 1 ou 2 groupes substituants sélectionnés dans le groupe consistant en un groupe méthyle et des atomes halogènes.

5. Utilisation du composé N-(2-[2-[(4-substitué phényl) amino]-2-oxoethyl] tétrahydro-2H-thiopyran-4-carboxamide représenté par la formule (I) décrit à la revendication 1, pour la fabrication d'un médicament destiné à la prévention ou au traitement de maladies causées par les virus de l'herpès résistants à l'acyclovir.
